# FASCICULE DE BREVET EUROPEEN

(11) **EP 1 010 978 B1**
(45) Date de publication et mention de la délivrance du brevet: **24.07.2002**
(21) Numéro de dépôt: 99403112.8
(22) Date de dépôt: 10.12.1999
(51) Int. Cl.: G01N 31/22, G01N 33/22

(54) **Indicateur coloré pour la mesure de la répartition des familles d'hydrocarbures**
Farbindikator zur Bestimmung der Verteilung von Kohlenwasserstoff
Colour indicator for measuring the distribution of hydrocarbons

(30) Priorité: 16.12.1998 FR 9815884
(43) Date de publication de la demande: 21.06.2000
(73) Titulaire: TOTAL RAFFINAGE DISTRIBUTION S.A., 92800 Puteaux (FR)
(72) Inventeur: Borg, Françoise, 92200 Neuilly sur Seine (FR); Seyfried, Luc, 76110 Ecrainville (FR); Lefebvre, Dominique, 76290 Montivilliers (FR)
(74) Mandataire: Jolly, Jean-Pierre

(56) Documents cités:
- WO-A-96/00271
- US-A- 5 487 770
- US-A- 5 827 332
- PATENT ABSTRACTS OF JAPAN vol. 015, no. 487 (P-1286), 10 décembre 1991 (1991-12-10) & JP 03 210472 A (COSMO SOGO KENKYUSHO:KK;OTHERS: 02), 13 septembre 1991 (1991-09-13)
- ZRELOV ET AL: "Oxidation liquid chromatography of fuel oils" CHEM.TECHNOL. FUEL OILS, vol. 16, no. 5-6, mai 1980 (1980-05), pages 356-360, XP002116264

## Description

La présente invention concerne un nouvel indicateur coloré permettant la mesure quantitative des différentes familles d'hydrocarbures présentes dans un mélange et, en particulier, dans une essence pour moteur à combustion interne et à allumage commandé, ou dans un carburéacteur, par la technique utilisant la rétention sélective de ces différentes familles de molécules sur un support adsorbant. L'invention concerne également un procédé d'obtention de ce nouvel indicateur coloré et ses utilisations.

### ETAT DE LA TECHNIQUE

On sait qu'une essence destinée à alimenter un moteur à combustion interne et à allumage commandé, est généralement constituée de différentes familles d'hydrocarbures, tels des hydrocarbures dits "saturés", comportant des liaisons simples entre les atomes de carbone, comme par exemple les molécules de type paraffinique ou naphténique, ainsi que des hydrocarbures appelés "insaturés", présentant des cycles benzéniques, ou au moins une double liaison entre les atomes de carbone, comme les molécules à structure aromatique ou oléfinique.

Ces différents hydrocarbures présents dans l'essence de l'automobiliste proviennent, pour l'essentiel, du pétrole brut à l'origine de la fabrication des carburants, ou résultent, comme pour les oléfines, des nombreuses transformations chimiques intervenant dans les différents procédés habituellement utilisés dans une raffinerie de pétrole, pour la fabrication de ces carburants. Les hydrocarbures saturés contenus dans une essence permettent d'améliorer, de manière non limitative :
- les propriétés de combustion dans les cylindres ;
- le rapport hydrogène/carbone pour augmenter le pouvoir calorifique du carburant ;
- la stabilité du carburant aux hautes températures ;
tandis que les hydrocarbures aromatiques et certains types d'oléfines favorisent l'augmentation de :
- l'indice d'octane pour éviter l'autoinflammation non contrôlée du carburant dans les cylindres ;
- la densité, pour améliorer le rendement du moteur tout en réduisant sa consommation.

On sait également que, devant les problèmes liés à l'environnement et notamment ceux posés par les émissions des différentes sources d'énergie fossile dans les villes, les législations déjà en vigueur et très certainement celles à venir, exigent un meilleur contrôle des quantités des familles d'hydrocarbures introduites dans les essences et, en particulier, des molécules susceptibles d'être préjudiciables à la qualité de l'air, donc à la santé des consommateurs.

Plusieurs méthodes analytiques sont disponibles pour contrôler qualitativement et/ou quantitativement la répartition des familles des molécules présentes dans un mélange hydrocarboné, comme par exemple :
- la spectrométrie de masse utilisant la méthode ASTM D2789, pour la mesure, par exemple, des composés aromatiques ;
- la chromatographie gazeuse et la méthode ASTM D5443, permettant de déterminer quantitativement les paraffines, les naphtènes, ainsi que les molécules aromatiques ;
- la chromatographie sur gel de silice, en application de la méthode ASTM D1319 (ou ISO 3837), pour la détermination des hydrocarbures aromatiques, oléfiniques et saturés.

Ces différentes méthodes permettent de mesurer les teneurs d'un ou plusieurs constituant(s) présent(s) dans un mélange hydrocarboné et, notamment, une essence pour moteur à combustion interne. Elles se différencient par leur précision, leur temps de mesure et aussi par leur complexité de mise en oeuvre, exigeant par exemple un spectromètre de masse ou une simple colonne de chromatographie.

Parmi ces méthodes, la plus utilisée aujourd'hui, la plus simple, la moins coûteuse, et surtout la seule spécifiée au niveau international à ce jour, est la chromatographie sur gel de silice, conformément à la méthode ASTM D1319. Cette méthode dite "FIA" pour "Fluorescent Indicator Adsorption", qui a été normalisée en 1954, permet de déceler et mesurer les quantités de molécules aromatiques contenues dans une essence dans la gamme de 5 % à 99 % en volume, des oléfines entre 0,3 % et 55 % en volume et tous les hydrocarbures saturés entre 1 % et 95 % en volume.

Aujourd'hui, cette technique analytique est retenue, dans le cadre de la révision de la méthode NF EN228, comme méthode d'analyse de référence dans les futures spécifications des essences pour moteurs à combustion interne, en préparation pour l'année 2000 et concernant plus particulièrement la détermination quantitative des oléfines et des hydrocarbures aromatiques présents dans les carburants.

La méthode FIA repose sur le principe de la chromatographie en phase liquide, technique selon laquelle l'essence ou le carburéacteur est élué par un alcool désorbant, comme par exemple l'isopropanol, sur une colonne remplie de silice. La séparation des composants est mise en évidence grâce à l'utilisation d'un indicateur coloré, constitué principalement de colorants jaune, bleu et rouge, spécifiques aux familles d'hydrocarbures, qui migrent dans la colonne sélectivement suivant la nature de ces hydrocarbures, et dont la coloration est détectable, en particulier pour les colorants jaune et bleu, de préférence sous une lumière de type ultraviolet.

La zone de la colonne correspondant aux hydrocarbures saturés est mesurée du front d'élution situé au bas de la colonne jusqu'au niveau de la coloration jaune la plus intense, tandis que la zone des oléfines se termine au niveau de la coloration bleue la plus intense indiquant le début de la zone des aromatiques, cette dernière étant limitée, en amont, par un anneau rouge.

La longueur de chaque zone ainsi identifiée sur la colonne est directement proportionnelle à la teneur en volume des molécules de type saturé, oléfinique ou aromatique, présentes dans le carburant, la somme de ces trois zones représentant 100 % des hydrocarbures présents dans le carburant.

Un problème posé par cette méthode analytique normalisée est qu'elle est mise en défaut et conduit à des résultats de mesures erronés, lorsque le carburant analysé contient, comme c'est de plus en plus souvent le cas dans les carburants actuellement commercialisés, certains produits oxygénés.

On sait en effet, qu'en vue d'ajuster l'indice d'octane des carburants, les raffineurs introduisent dans ceux-ci des composés dits oxygénés, sous forme d'alcools, tels que le méthanol ou l'éthanol, ou sous forme d'éthers, comme le méthyl-tert butyléther (MTBE), l'éthyl-tert-butyléther (ETBE), ou encore le di-isopropyl éther (DIPE), ou le tert-amylméthyléther (TAME), ou des mélanges de ces composés oxygénés, qui évitent des risques de pollution à l'échappement des véhicules, dus aux dérivés organiques du plomb préalablement utilisés et dont l'interdiction d'utilisation est prévue en Europe à partir de l'an 2000, sauf dérogations qui pourraient être accordées à certains pays.

La méthode ASTM D1319, qui a connu deux révisions en 1995, indique qu'aux concentrations normalement utilisées dans les essences commerciales et qui varient usuellement entre 5 % et 20 % en volume, ces composés oxygénés introduits dans les essences n'interfèrent pas avec la détermination des familles d'hydrocarbures. Ces composés oxygénés ne sont pas détectés par la méthode FIA, car, éluant avec l'alcool désorbant, ils n'auraient par conséquent pas d'influence sur la répartition des hydrocarbures dans la colonne chromatographique et donc sur leur mesure.

### EXPOSE DETAILLE DE L'INVENTION

Au cours de ses travaux dans le domaine du contrôle par la méthode FIA de la composition des carburants pour moteur à combustion interne et à allumage commandé, la Demanderesse a mis en évidence, à l'aide d'essais qui seront exposés dans la suite de la présente description, qu'une analyse réalisée par la méthode FIA sur un carburant contenant des produits oxygénés, notamment un éther, tel que l'ETBE, conduit à des écarts considérables avec les teneurs en hydrocarbures préalablement obtenues sur le même carburant sans produits oxygénés, quand on applique strictement la méthode ASTM D1319.

Il apparaît donc que la présence de l'ETBE et d'autres composés oxygénés comme, par exemple, le TAME et le DIPE, seuls ou en mélange dans les essences avec les hydrocarbures, est responsable d'une erreur systématique sur la mesure des teneurs de certains constituants d'une essence par la méthode FIA, quand on applique strictement la méthode ASTM D1319, la teneur en hydrocarbures aromatiques étant surestimée tandis que la teneur en hydrocarbures saturés est sous-estimée.

Plus précisément, la Demanderesse a établi que les produits oxygénés présents dans les mélanges d'hydrocarbures à analyser par la méthode FIA et dont la molécule comprend au moins 6 atomes de carbone se comportent comme des hydrocarbures aromatiques au cours de l'élution et expliquent les écarts enregistrés par cette analyse, en particulier quand les mélanges contiennent de l'ETBE.

L'indicateur coloré proposé aujourd'hui dans la méthode et disponible dans le commerce n'est donc pas adapté à tous les types de carburant et notamment à ceux qui contiennent, par exemple, de l'ETBE.

Des améliorations de ces indicateurs ont certes été proposées, comme dans DE-A-2 421 793, pour augmenter leur lisibilité sous lampe ultraviolette, mais non pour les rendre indépendants de la composition du carburant analysé.

L'invention vise donc à proposer un indicateur coloré, utilisable pour la détermination des familles d'hydrocarbures présentes dans un mélange, par la technique d'analyse utilisant la chromatographie en phase liquide sur colonne, comme, par exemple, celle enseignée dans la méthode ASTM D1319, ou équivalente, dont la réponse est indépendante de la constitution de l'échantillon et plus précisément de la présence de certains composés oxygénés couramment utilisés, seuls ou en mélanges, dans les essences pour moteur à combustion interne.

A cet effet, l'invention a pour premier objet un indicateur coloré pour la mesure par chromatographie en phase liquide sur colonne des teneurs en composés hydrocarbonés appartenant aux familles des hydrocarbures saturés, oléfiniques et aromatiques, présents dans un mélange, qui contient par ailleurs des composés oxygénés possédant au moins six atomes de carbone, cet indicateur coloré contenant des quantités substantielles de divers colorants susceptibles d'être élués sélectivement avec chacune de ces familles en exprimant des couleurs différentes pour chacune de celles-ci, et étant caractérisé en ce que les colorants susceptibles d'être élués avec la famille des hydrocarbures aromatiques sont dépourvus de composés organiques ayant une affinité avec les composés oxygénés comprenant au moins 6 atomes de carbone.

L'indicateur coloré conforme à l'invention peut être dérivé de n'importe quel type d'indicateur coloré recommandé pour analyse chromatographique et, en particulier, pour l'application de la méthode ASTM D1319, par exemple, du type commercialisé sous l'appellation "Fluorescent Indicator", par les sociétés Universal Oil Products ou Merck.

Cet indicateur coloré disponible dans le commerce est constitué, par exemple, d'un gel coloré composé d'un mélange du colorant dénommé "Petrol Red AB4" qui a été recristallisé, et de fractions de colorants oléfiniques et aromatiques (jaunes et bleues) purifiées et déposées sur un gel de silice.

Un colorant rouge pouvant également être utilisé dans l'indicateur coloré pour FIA et bien connu de l'homme du métier pour être facilement élué par des hydrocarbures aromatiques est commercialisé sous l'appellation "Sudan 3" ou "Sudan 4", et appartient à la famille des colorants azoïques aromatiques et, plus généralement, à la famille des composés hydroxy arylés qui sont obtenus, par exemple pour le Sudan 3 (C₂₂ H₁₆ N₄ O), par réaction de copulation entre un dérivé de déazotation et un copulant de type amine aromatique.

La Demanderesse a établi que les colorants destinés à être élués par les hydrocarbures aromatiques présents, par exemple dans les carburants, sont constitués, comme dans le cas du Sudan 3, d'un mélange de pigments rouge et brun, le pigment rouge étant élué par les composés aromatiques et les composés oxygénés comportant au moins 6 atomes de carbone, tandis que le pigment brun est élué seulement par les composés aromatiques. C'est ce pigment brun qui est seul retenu par la Demanderesse et qui est inclus dans le nouvel indicateur coloré pour être élué spécifiquement par les molécules aromatiques présentes, par exemple, dans un carburant ou un carburéacteur.

Bien entendu, l'indicateur coloré conforme à l'invention peut contenir tous types de colorants susceptibles d'être élués avec des hydrocarbures aromatiques, si ces colorants ne présentent pas d'affinité pour les produits oxygénés contenant au moins 6 atomes de carbone et, notamment, l'ETBE, le TAME, le DIPE, utilisés seuls ou en mélanges.

L'invention a par conséquent pour second objet un procédé de préparation d'un indicateur coloré du type défini ci-dessus, à partir d'un indicateur coloré d'un type connu, utilisé pour la détermination par chromatographie en phase liquide sur colonne des familles d'hydrocarbures contenues dans un mélange, ce procédé étant caractérisé en ce que l'on extrait de l'indicateur connu, à l'aide d'au moins un composé oxygéné contenant au moins 6 atomes de carbone, les colorants organiques susceptibles d'être élués par les composés oxygénés comprenant au moins 6 atomes de carbone.

De façon plus précise, on élue par exemple l'indicateur coloré connu, recommandé par la méthode FIA, sur une colonne chromatographique, à l'aide d'un composé oxygéné comprenant au moins 6 atomes de carbone, et l'on sépare et recueille séparément les différents constituants de cet indicateur coloré, on élue à nouveau le colorant rouge ainsi séparé sur une colonne chromatographique avec un composé oxygéné comprenant au moins 6 atomes de carbone, qui peut être identique à celui de la première élution, en vue de séparer ce colorant rouge en deux fractions, l'une rouge et l'autre brune, on recueille la fraction brune et on la mélange intimement avec les colorants jaune et bleu précédemment séparés, pour former l'indicateur coloré conforme à l'invention.

Le composé oxygéné à au moins 6 atomes de carbone utilisé dans ce procédé est de préférence choisi dans le groupe comprenant l'ETBE, le TAME, le DIPE ou leurs mélanges.

Une autre forme d'extraction du colorant rouge de l'indicateur coloré d'un type connu comprend le mélange sous agitation de cet indicateur coloré avec des produits oxygénés comportant au moins 6 atomes de carbone, le colorant rouge ainsi séparé et recueilli dans la phase organique étant ensuite élué dans une colonne chromatographique en vue de sa séparation de la manière indiquée ci-dessus.

Avantageusement, pour améliorer la séparation du colorant rouge présent dans l'indicateur coloré connu de départ, on utilise un solvant aromatique tel que le toluène, auquel est ajouté le composé oxygéné contenant au moins 6 atomes de carbone.

L'indicateur coloré conforme à l'invention peut par conséquent être préparé à partir d'un colorant rouge disponible dans le commerce, par exemple le Sudan 3 ou le Sudan 4, à partir duquel est extrait, par exemple par élution, la fraction brune, cette fraction brune étant ensuite mélangée avec les colorants jaune et bleu.

La présente invention a enfin pour objet l'utilisation de l'indicateur coloré conforme à l'invention pour la mesure de la répartition des familles d'hydrocarbures contenues dans un mélange, par une variante de la méthode FIA selon la méthode ASTM D1319, selon laquelle la lecture de la colonne chromatographique s'effectue de façon usuelle, en se référant à des anneaux respectivement jaune et bleu, pour les hydrocarbures saturés et oléfiniques, cette utilisation étant caractérisée en ce que, pour déceler la fin des composés aromatiques, on prend pour référence sur la colonne chromatographique un anneau brun et non plus rouge, le pigment brun de l'indicateur coloré étant élué spécifiquement par les hydrocarbures aromatiques.

Les exemples ci-après illustrent de manière non limitative, l'invention.

### Exemple 1

Cet exemple est destiné à illustrer les erreurs apparaissant dans les résultats de la mesure par la méthode FIA, selon la méthode ASTM D1319, de la répartition des familles d'hydrocarbures dans un mélange, par chromatographie en phase liquide à l'aide d'un indicateur coloré du commerce, lorsque le mélange d'hydrocarbures contient un composé oxygéné tel qu'un éther, à au moins 6 atomes de carbone.

Dans ce but, la Demanderesse a utilisé une essence du commerce, portant la référence SP95, dont elle a effectué l'analyse en appliquant strictement la méthode ASTM D1319, en utilisant l'indicateur coloré commercialisé par la société Universal Oil Products.

Elle a ensuite procédé aux mêmes mesures sur le même carburant additionné de 15% en volume d'ETBE.

Les résultats obtenus sont rassemblés dans le Tableau 1 ci-après.

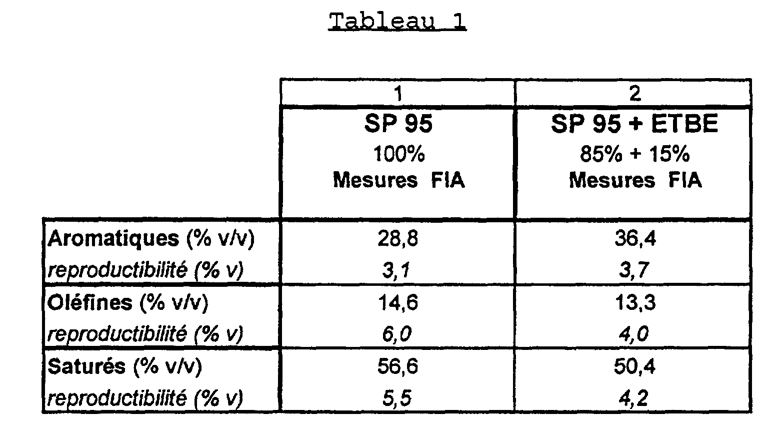

Dans ce tableau, les teneurs sont exprimées en prenant pour base un total de 100 % d'hydrocarbures aromatiques, d'hydrocarbures oléfiniques et d'hydrocarbures saturés.

On voit sur le Tableau 1 (colonne 2) que l'application de la méthode FIA, c'est à dire la mesure des familles d'hydrocarbures contenues dans un carburant, par la technique de chromatographie en phase liquide sur colonne, conduit à des résultats erronés, lorsque l'essence analysée contient de l'ETBE. En effet, des écarts importants apparaissent sur ces résultats, quand la mesure est réalisée en application de la méthode ASTM-D1319:
- écart positif et significatif (+7,6 %) sur la mesure des hydrocarbures aromatiques,
- écart négatif et significatif (-6,2 %) sur la mesure des hydrocarbures saturés.

### Exemple 2

En vue de mieux mettre en évidence l'incidence de la présence de composés oxygénés sur les réponses FIA, la Demanderesse a réalisé des mesures FIA sur deux catégories de composés oxygénés purs tels que définis dans le Tableau 2 et habituellement rencontrés dans les essences pour moteurs à combustion interne et à allumage commandé.

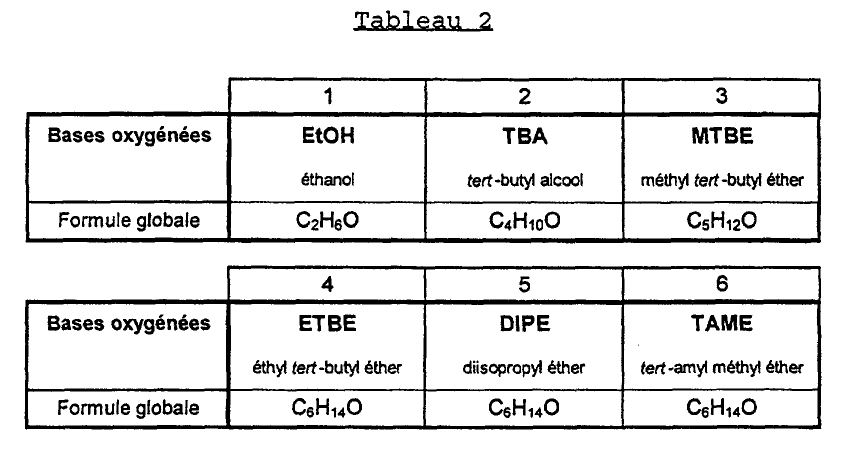

Ces essais ont apporté les enseignements suivants :
- pour les composés oxygénés légers comportant par exemple de 2 à 5 atomes de carbone (colonnes 1,2 et 3), comme l'éthanol, le TBA (tert-butyl alcool) ou le MTBE, aucune réponse FIA n'est attribuable à une famille d'hydrocarbures. Les colorants jaune, bleu et rouge, spécifiques des différentes familles d'hydrocarbures présentes dans le carburant, sont élués régulièrement et traversent la colonne à des vitesses sensiblement comparables, pour se situer au niveau du front d'élution, au bas de la colonne, traduisant ainsi une absence de réponse FIA ;
- pour les oxygénés plus lourds avec au moins 6 atomes de carbone (colonnes 4,5 et 6) comme par exemple l'ETBE, le DIPE ou le TAME, les colorants jaune et bleu migrent régulièrement comme précédemment, pour se stabiliser sur le front d'élution au bas de la colonne, tandis que le colorant rouge progresse plus lentement et reste en queue de colonne, en place de la limite haute des aromatiques. Selon l'application de la méthode ASTM D1319, cette mesure à l'anneau rouge est équivalente à une réponse correspondant à 100% de molécules aromatiques.

Il apparaît donc que, contrairement à ce qu'énonce la méthode ASTM D1319, l'ETBE, le DIPE et le TAME produisent une réponse, lorsque l'on applique la méthode FIA, en raison d'une élution par ces produits oxygénés du colorant rouge (anneau rouge) de l'indicateur coloré préconisé par la méthode, comme le font les hydrocarbures aromatiques. Les produits oxygénés dont la molécule contient 6 atomes de carbone se comportent donc comme des hydrocarbures aromatiques et expliquent les écarts enregistrés sur les résultats de la méthode FIA, quand le carburant analysé contient par exemple de l'ETBE.

### Exemple 3

On effectue des essais d'analyse par la méthode FIA, dans les conditions de la méthode ASTM D1319, en utilisant comme mélange d'hydrocarbures une essence (essence A) pour moteur à combustion interne et à allumage commandé, seule ou additionnée de diverses quantités de composés oxygénés, en utilisant comme indicateur coloré :
- l'indicateur coloré commercialisé sous l'appellation "Fluorescent Indicator", par la société Universal Oil Products ;
- l'indicateur coloré conforme à l'invention, obtenu à partir du précédent par extraction du colorant rouge qu'il contient.

Avec l'indicateur coloré conforme à l'invention, la lecture de la fin de zone des composés aromatiques sur la colonne de chromatographie est effectuée sur l'anneau brun, puisque l'indicateur utilisé ne comporte plus de colorant rouge.

Les résultats obtenus sont rassemblés dans le Tableau 3 ci-après.

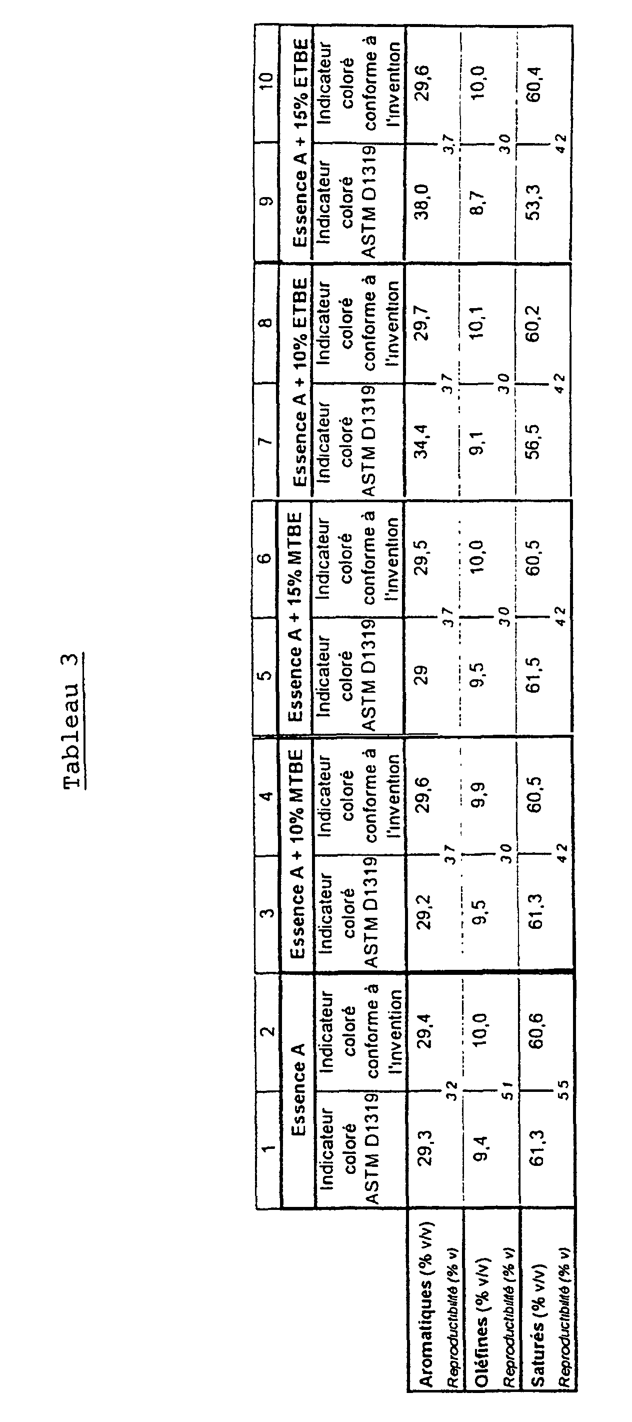

Ces résultats montrent clairement que l'indicateur coloré conforme à l'invention permet d'effectuer des mesures fiables par la méthode FIA, quelles que soient la nature et la quantité des composés oxygénés présents, dans le mélange à analyser (colonnes 2, 4, 6, 8 et 10), ce qui n'est pas le cas de l'indicateur coloré préconisé par la méthode ASTM D1319, quand le carburant contient un composé oxygéné avec au moins 6 atomes de carbone (colonnes 7 et 9).

## Revendications

1. Indicateur coloré pour la mesure par chromatographie en phase liquide sur colonne des teneurs en composés hydrocarbonés appartenant aux familles des hydrocarbures saturés, oléfiniques et aromatiques, présents dans un mélange, qui contient par ailleurs des composés oxygénés possédant au moins six atomes de carbone, cet indicateur coloré contenant des quantités substantielles de divers colorants susceptibles d'être élués sélectivement avec chacune de ces familles en exprimant des couleurs différentes pour chacune de celles-ci, et étant **caractérisé en ce que** les colorants susceptibles d'être élués avec la famille des hydrocarbures aromatiques sont dépourvus de composés organiques ayant une affinité avec les composés oxygénés comprenant au moins 6 atomes de carbone.

2. Indicateur selon la revendication 1, caractérisé en ce les colorants susceptibles d'être élués sélectivement avec la famille des hydrocarbures aromatiques appartiennent à la famille des composés hydroxy arylés.

3. Indicateur coloré selon la revendication 2, **caractérisé en ce que** les colorants susceptibles d'être élués sélectivement avec la famille des hydrocarbures aromatiques sont des colorants de type azoïque aromatique.

4. Indicateur coloré selon l'une des revendications 1 à 3, **caractérisé en ce qu'**il comprend un indicateur coloré d'un type connu en soi préconisé pour la mise en oeuvre de la méthode d'analyse selon la norme ASTM D1319, dont ont été éliminés les colorants susceptibles d'être élués avec la famille des hydrocarbures aromatiques et ayant une affinité avec les composés oxygénés comprenant au moins 6 atomes de carbone.

5. Indicateur coloré selon la revendication 4, **caractérisé en ce qu'**il est dépourvu du colorant rouge présent dans l'indicateur coloré connu préconisé pour la mise en oeuvre de la méthode d'analyse selon la norme ASTM 1319, mais non du pigment brun présent également dans cet indicateur connu.

6. Procédé de préparation d'un indicateur coloré selon l'une des revendications 1 à 5, à partir d'un indicateur coloré d'un type connu, utilisé pour la détermination par chromatographie en phase liquide sur colonne des familles d'hydrocarbures contenues dans un mélange, ce procédé étant **caractérisé en ce que** l'on extrait de l'indicateur connu, à l'aide d'au moins un composé oxygéné contenant au moins 6 atomes de carbone, les colorants organiques susceptibles d'être élués par les composés oxygénés comprenant au moins 6 atomes de carbone.

7. Procédé selon la revendication 6, **caractérisé en ce que** le composé oxygéné contenant au moins 6 atomes de carbone est choisi dans le groupe comprenant l'ETBE, le TAME, le DIPE et leurs mélanges.

8. Procédé selon l'une des revendications 6 et 7, **caractérisé en ce que** l'on élue sur une colonne chromatographique à l'aide d'un composé oxygéné comprenant au moins 6 atomes de carbone, un indicateur coloré préconisé pour la mise en oeuvre de la méthode d'analyse selon la norme ASTM D1319, **en ce que** l'on sépare et recueille séparément les colorants jaune, bleu et rouge de cet indicateur, **en ce que** l'on élue à nouveau le colorant rouge ainsi séparé sur une colonne chromatographique avec un composé oxygéné comprenant au moins 6 atomes de carbone, qui peut être identique à celui de la première élution, en vue de séparer ce colorant rouge en deux fractions, l'une rouge et l'autre brune, **en ce que** l'on recueille la fraction brune et **en ce qu'**on la mélange intimement avec les colorants jaune et bleu précédemment séparés, pour former l'indicateur coloré désiré.

9. Procédé selon la revendication 6, **caractérisé en ce que** l'extraction de l'indicateur coloré d'un type connu des colorants organiques susceptibles d'être élués par les composés organiques comprenant au moins 6 atomes de carbone s'effectue par mélange sous agitation de cet indicateur coloré connu avec au moins un produit oxygéné comportant au moins 6 atomes de carbone.

10. Procédé selon l'une des revendications 6 à 9, **caractérisé en ce que** le produit oxygéné à au moins 6 atomes de carbone est utilisé en mélange avec un solvant aromatique, tel que le toluène.

11. Utilisation de l'indicateur coloré selon l'une des revendications 1 à 5, pour la mesure de la répartition des familles d'hydrocarbures contenues dans un mélange, par une variante de la méthode FIA selon la norme ASTM D1319, dans laquelle la lecture de la colonne chromatographique s'effectue de façon usuelle, en se référant à des anneaux respectivement jaune et bleu, pour les hydrocarbures saturés et oléfiniques, cette utilisation étant **caractérisée en ce que**, pour déceler la fin des composés aromatiques, on prend pour référence sur la colonne chromatographique un anneau brun et non plus rouge, le pigment brun de l'indicateur coloré étant élué spécifiquement par les hydrocarbures aromatiques.

12. Utilisation selon la revendication 11, pour l'analyse d'un mélange dans lequel est en outre présent au moins un composé oxygéné du type éther à au moins 6 atomes de carbone.

## Patentansprüche

1. Farbindikator zur säulenchromatographischen Bestimmung in flüssiger Phase des jeweiligen Gehalts an Kohlenwasserstoffverbindungen, welche zu Gruppen gesättigter, olefinhaltiger und aromatischer Kohlenwasserstoffe gehören, die in einem Gemisch vorliegen, welches außerdem sauerstoffhaltige Verbindungen mit mindestens sechs Kohlenstoffatomen enthalten, wobei dieser Farbindikator verschiedene Farbstoffe in erheblichen Mengen enthält, die zur selektiven Elution mit jeder dieser Gruppen geeignet sind, wobei sie für jede derselben unterschiedliche Farben zeigen, **dadurch gekennzeichnet, dass** die mit der Gruppe der aromatischen Kohlenwasserstoffe eluierbaren Farbstoffe arm an organischen Verbindungen mit Affinität zu den sauerstoffhaltigen Verbindungen sind, die mindestens 6 Kohlenstoffatome besitzen.

2. Farbindikator nach Anspruch 1, **dadurch gekennzeichnet, dass** die selektiv mit der Gruppe der aromatischen Kohlenwasserstoffe eluierbaren Farbstoffe zur Gruppe der arylierten Hydroxy-Verbindungen gehören.

3. Farbindikator nach Anspruch 2, **dadurch gekennzeichnet, dass** die selektiv mit der Gruppe der aromatischen Kohlenwasserstoffe eluierbaren Farbstoffe Färbemittel von der Art aromatischer Azo-Verbindungen sind.

4. Farbindikator nach einem der Ansprüche 1 bis 3, **dadurch gekennzeichnet, dass** er einen Farbindikator an sich bekannter Art enthält, der zur Durchführung des Analyseverfahrens nach der Norm ASTM D1319 vorgesehen ist und bei dem die Farbstoffe herausgelöst wurden, die mit der Gruppe der aromatischen Kohlenwasserstoffe eluierbar sind und eine Affinität gegenüber den sauerstoffhaltigen Verbindungen aufweisen, die mindestens 6 Kohlenstoffatome besitzen.

5. Farbindikator nach Anspruch 4, **dadurch gekennzeichnet, dass** er arm an rotem Farbstoff ist, der in dem bekannten Farbindikator vorhanden ist, welcher für die Durchführung des Analyseverfahrens nach der Norm ASTM D1319 vorgesehen ist, aber keinen brauen Farbstoff, der ebenfalls in diesem bekannten Indikator enthalten ist.

6. Verfahren zur Herstellung eines Farbindikators nach einem der Ansprüche 1 bis 5, aus einem Farbindikator bekannter Art, der zur säulenchromatographischen Bestimmung in flüssiger Phase von Gruppen von Kohlenwasserstoffen eingesetzt wird, die in einem Gemisch enthalten sind, **dadurch gekennzeichnet, dass** aus dem bekannten Indikator mit Hilfe mindestens einer sauerstoffhaltigen Verbindung extrahiert wird, welche mindestens 6 Kohlenstoffatome aufweist, die organischen Farbstoffe herausgelöst werden, die durch die sauerstoffhaltigen Verbindungen mit mindestens 6 Kohlenstoffatomen eluierbar sind.

7. Verfahren nach Anspruch 6, **dadurch gekennzeichnet, dass** die sauerstoffhaltige Verbindung, die mindestens 6 Kohlenstoffatome enthält, aus der Gruppe gewählt wird, die ETBE, TAME, DIPE und deren Gemische umfasst.

8. Verfahren nach einem der Ansprüche 6 und 7, **dadurch gekennzeichnet, dass** auf einer Chromatographiesäule mit Hilfe einer sauerstoffhaltigen Verbindung mit mindestens 6 Kohlenstoffatomen ein Farbindikator eluiert wird, der zur Durchführung des Analyseverfahrens nach der Norm ASTM D1319 vorgesehen ist, dass diese abgetrennt wird und man die gelben, blauen und roten Farbstoffe dieses Indikators getrennt auffängt, dass der auf diese Weise abgetrennte rote Farbstoff erneut auf einer Chromatographiesäule mit einer sauerstoffhaltigen Verbindung eluiert wird, die mindestens 6 Kohlenstoffatome enthält und welche identisch mit der beim ersten Eluierungsschritt verwendeten Verbindung sein kann, um so diesen roten Farbstoff in zwei Fraktionen aufzutrennen, nämlich eine rote und eine braune, dass die braune Fraktion aufgefangen wird und dass das Gemisch innig mit den zuvor getrennten gelben und blauen Farbstoffen vermischt wird, um so den gewünschten Farbindikator zu bilden.

9. Verfahren nach Anspruch 6, **dadurch gekennzeichnet, dass** die Herauslösung der organischen Farbstoffe, die mit den organischen Verbindungen eluierbar sind, welche mindestens 6 Kohlenstoffatome enthalten, aus dem Farbindikator bekannter Art durch Vermischen dieses bekannten Farbindikators unter Rühren mit mindestens einem sauerstoffhaltigen Produkt durchgeführt wird, welches mindestens 6 Kohlenstoffatome enthält.

10. Verfahren nach einem der Ansprüche 6 bis 9, **dadurch gekennzeichnet, dass** das sauerstoffhaltige Produkt mit mindestens 6 Kohlenstoffatomen in Mischung mit einem aromatischen Lösungsmittel wie beispielsweise Toluol verwendet wird.

11. Verwendung des Farbindikators nach einem der Ansprüche 1 bis 5 zur Bestimmung der Verteilung von Kohlenwasserstoffgruppen, die in einem Gemisch enthalten sind, unter Einsatz einer Variante des FIA-Verfahrens gemäß der Norm ASTM D1319, bei welchem bei gesättigten und olefinhaltigen Kohlenwasserstoffen die Ablesung der Chromatographiesäule in der üblichen Weise erfolgt, wobei jeweils auf einen gelben und einem blauen Ring Bezug genommen wird, **dadurch gekennzeichnet, dass** zum Nachweis des Endes der aromatischen Verbindungen auf der Chromatographiesäule ein brauner Ring und nicht mehr ein roter Ring als Referenz herangezogen wird, wobei der braune Farbstoff des Farbindikators spezifisch durch die aromatischen Kohlenwasserstoffe eluiert wird.

12. Verwendung nach Anspruch 11 zur Analyse eines Gemisches, in welchem außerdem mindestens eine sauerstoffhaltige Verbindung in der Art von Ether mit mindestens 6 Kohlenstoffatomen vorhanden ist.

## Claims

1. A coloured indicator for measurement by liquid phase column chromatography of the contents of hydrocarbon compounds belonging to the families of saturated, olefinic and aromatic hydrocarbons present in a mixture which moreover contains oxygenates having at last six carbon atoms, this coloured indicator containing substantial quantities of various dyes capable of being eluted selectively with each of these families, exhibiting different colours for each of said families, and being **characterised in that** the dyes capable of being eluted with the family of aromatic hydrocarbons are free of any organic compounds having an affinity with oxygenates comprising at least 6 carbon atoms.

2. An indicator according to claim 1, **characterised in that** the dyes capable of being eluted selectively with the family of aromatic hydrocarbons belong to the family of hydroxyaryl compounds.

3. A coloured indicator according to claim 2, **characterised in that** the dyes capable of being eluted selectively with the family of aromatic hydrocarbons are dyes of the aromatic azo type.

4. A coloured indicator according to one of claims 1 to 3, **characterised in that** it comprises a coloured indicator of a type known *per se* recommended for performance of the analytical method according to standard ASTM D1319, from which have been removed the dyes capable of being eluted with the family of aromatic hydrocarbons and having an affinity with oxygenates comprising at least 6 carbon atoms.

5. A coloured indicator according to claim 4, **characterised in that** it is free of the red dye present in the known coloured indicator recommended for performance of the analytical method according to standard ASTM 1319, but not of the brown pigment also present in said known indicator.

6. A process for the preparation of a coloured indicator according to one of claims 1 to 5, starting from a coloured indicator of known type, used for determining the families of hydrocarbons contained in a mixture by liquid phase column chromatography, said process being **characterised in that** the organic dyes capable of being eluted by oxygenates comprising at least 6 carbon atoms are extracted from the known indicator by means of at least one oxygenate containing at least 6 carbon atoms.

7. A process according to claim 6, **characterised in that** the oxygenate containing at least 6 carbon atoms is selected from the group comprising ETBE, TAME, DIPE and mixtures thereof.

8. A process according to one of claims 6 and 7, **characterised in that** a coloured indicator recommended for performance of the analytical method according to standard ASTM D1319 is eluted on a chromatographic column by means of an oxygenate comprising at least 6 carbon atoms; **in that** the yellow, blue and red dyes of this indicator are separated and collected separately, **in that** the red dye separated in this manner is eluted again on a chromatographic column with an oxygenate comprising at least 6 carbon atoms, which may be identical to that from the first elution, with the aim of separating this red dye into two fractions, one red and one brown, **in that** the brown fraction is collected and **in that** said fraction is mixed intimately with the previously separated yellow and blue dyes to constitute the desired coloured indicator.

9. A process according to claim 6, **characterised in that** extraction of the coloured indicator of known type from the organic dyes capable of being eluted by organic compounds comprising at least 6 carbon atoms is performed by mixing said known coloured indicator with shaking with at least one oxygenate comprising at least 6 carbon atoms.

10. A process according to one of claims 6 to 9, **characterised in that** the oxygenate comprising at least 6 carbon atoms is used as a mixture with an aromatic solvent, such as toluene.

11. Use of a coloured indicator according to one of claims 1 to 5 for measuring the distribution of the families of hydrocarbons contained in a mixture by means of a variant of the FIA method according to standard ASTM D1319, in which the chromatographic column is read in the conventional manner with reference to yellow and blue rings for the saturated and olefinic hydrocarbons respectively, said use being **characterised in that**, in order to detect the end of the aromatic compounds, a brown ring on the chromatographic column is now used as the reference instead of a red ring, the brown pigment of the coloured indicator being specifically eluted by aromatic hydrocarbons.

12. Use according to claim 11 for the analysis of a mixture which furthermore contains at least one ether-type oxygenate having at least 6 carbon atoms.
